# EUROPEAN PATENT APPLICATION

(11) **EP 4 461 858 A1**
(43) Date of publication of application: **13.11.2024**
(21) Application number: 24174908.4
(22) Date of filing: 08.05.2024
(51) Int. Cl.: D04H 1/425, D01D 5/08, D01F 1/10, D01F 6/46, D04H 1/544, D04H 1/74, D04H 1/541, A61F 13/511, A61F 13/514, A61L 15/34, A61K 8/02, D01D 5/098

(54) **CARDED CALENDERED NONWOVENS**

(30) Priority: 11.05.2023 WO PCT/CN2023/093447; 11.05.2023 WO PCT/CN2023/093458
(71) Applicant: The Procter & Gamble Company, Cincinnati, OH 45202 (US); Indorama Ventures Public Company Ltd, 10110 Bangkok (TH)
(72) Inventor: LINDNER, Torsten, 65824 Schwalbach am Taunus (DE); ERDEM, Gueltekin, Cincinnati, 45202 (US); SAEVECKE, Dirk, 65824 Schwalbach am Taunus (DE); DESAI, Prashant, 10110 Bangkok (TH); DAHRINGER, Jörg Thomas, 86399 Bobingen (DE); KLINT, Søren Tang, 6800 Varde (DK); GUTMANN, Patrick, 86399 Bobingen (DE); BERGMANN, Mads Rudbeck, 6800 Varde (DK)
(74) Representative: P&G Patent Germany

(57) **Abstract**

A carded calendered nonwoven comprising synthetic staple fibers, wherein the synthetic staple fibers comprise a polypropylene polymer matrix, an ethylenepropylene copolymer and a fatty acid amide. The nonwovens may be used in application where a soft and affordable nonwoven is desired, for example disposable absorbent articles such as diapers.

## Description

### FIELD OF THE INVENTION

The invention relates to carded calendered nonwovens comprising staple synthetic fibers and optionally natural fibers. The nonwovens may be used in applications where a soft and affordable nonwoven is desired, for example disposable absorbent articles such as diapers. The invention also relates to the staple fibers that can be used to make the carded nonwovens.

### BACKGROUND OF THE INVENTION

Absorbent articles for personal hygiene such as diapers for babies or incontinent adults are designed to absorb and contain body exudates, in particular urine. These absorbent articles typically comprise several layers, in particular a fluid permeable topsheet on the body-facing side, a fluid impermeable backsheet on the garment-facing side and an absorbent core sandwiched in-between these layers. Pant-shaped products also typically comprise an inner and outer nonwoven belt.

The topsheet is directly in contact with the skin for a prolonged time. The backsheet is handled by the caregiver before the article is placed on the wearer. It is usually desirable that these external layers of absorbent articles are soft to the touch.

Nonwovens are commonly used in disposable absorbent articles. Nonwovens are sheet or web structures bonded together by entangling fibers or filaments mechanically, thermally, or chemically. They are flat, porous sheets that are made directly from separate fibers. They are not made by weaving or knitting and do not require converting the fibers to yarn. Common process to make nonwovens are meltblowing, spunbonding, solvent spinning, electrospinning, carding and airlaying. The basis weight of nonwoven webs is usually expressed in grams per square meter (g/m² or gsm).

Nonwovens are affordable, safe and versatile. They can display a variety of properties including softness, liquid barrier properties or alternatively liquid acquisition properties, depending on their structure and any post-treatments. A topsheet may be typically formed by a soft nonwoven which is easily wettable. A backsheet is typically a laminate of a liquid barrier plastic film and a nonwoven outer cover having a soft feel.

Spunlaid nonwovens are made in one continuous process where molten polymer granules are extruded into filaments through so called spinnerets. The continuous filaments are stretched and quenched before being deposited on a conveyor belt to form a uniform web. Spunlaid nonwovens have an increased strength compared to carded nonwovens, due to the attenuation of the filaments. The downside is that the choice of raw materials is more restricted. The term "spunbonded nonwoven" designates thermo bonded spunlaid.

Carded nonwovens on the other hand are made from staple fibers that are mechanically processed. Staple fibers are fibers having a discrete length which is much smaller than spunlaid fibers. In a typical carding process, bales of staple fibers are brought to a carding line where the bales are unwrapped. The bales are opened at a bale opener and the fibers are initially separated and transported by conveyer belt to the fiber preparation step where further fiber opening is taking place. The staple fibers then enter a master chute where the web going into the card is prepared. The master chute is also controlling the dosing of the fibers and ensures that there is an even distribution of fibers throughout the width of the web. Inside the card, the fibers are distributed in both the machine direction, MD, and cross direction, CD, depending on several parameters such as line speed, fiber cohesion and randomizers amongst other. The carded web obtained has limited initial strength and needs therefore to be consolidated. Consolidation is typically carried by air-through bonding or calendering bonded.

Air-through bonding (ATB) is a type of thermal bonding that uses a hot air stream to melt bicomponent fibers mixed with the staple fibers. The bicomponent fibers typically comprise Polyethylene (PE) in the sheath and Polypropylene (PP) in the core. PE has a lower melting temperature and junction points can thus be created by fusing the PE component fibers of individual component fibers, including PE mono-fibers. Air-through bonded carded nonwovens typically have excellent softness. ATB is however a comparably costly approach as it requires relatively expensive bicomponent fibers and uses a high energy consumption at a comparably low line speed.

In contrast to ATB, calendering is a different thermal bonding method that uses heat and pressure applied between two heated rollers to weld the staple fibers together at high speed. The bottom roller may be smooth, and the top roller may be engraved with a pattern, providing the desired bonding characteristics for the final nonwoven. The two rollers are pressed together and the combination of pressure and heat results in thermal bonding points where the two rollers are touching. These bonding points in the final nonwoven are what gives the tensile properties, for both MD and CD, as well as influence the softness of the nonwoven. As a general rule, a high bonding area results in lower softness. After the consolidation step, the obtained carded nonwoven can be wound up on large rolls.

Calendered carded nonwovens are typically cheaper to produce than ATB nonwovens. The calendering process can be run at typically 2x to 3x higher line speed than the air-through bonding process. The calendering process also requires significantly less energy, as only the bond points need to be heated rather than the whole web structure. Calendered carded webs can be run with mono fibers made from polypropylene (PP). PP is preferably used for mono-fibers because of its known better fiber spinning behavior. PP also typically has a lower cost versus polyethylene (PE). Calendered nonwovens however are generally less soft due to the presence of the macroscopic thermo-bonding pattern, with plastic-type hard bonding points. This diminishes the textile character of the calendered nonwoven. Calendered carded nonwovens can be in principle also made from bicomponent fibers, but the resulting webs are known to be less strong than calendered carded webs made from mono-component fibers. Therefore, mono fibers are also preferred for performance reasons in the context of calendered carded nonwovens.

Furthermore, the bicomponent fibers used in ATB carded nonwovens have typically a sheath of PE (as the lower melting point component) and a core of PP (as the higher melting point component). The PE sheath further contributes to enhance the softness impression of the web, presumably since PE has a lower flexural modulus than PP. In comparison with PP spunbond filaments, PP staple fibers have a higher crystallinity due to the drawing or stretching step which is part of the staple fiber making process. Thus, PP staple fibers have a higher stiffness, which is disadvantageous for the softness impression.

Looking at all these effects combined (plastic-like bond points in calendered bonded webs, use of PP as most preferred polymer to make the mono fibers, higher crystallinity in staple fibers than in spunbond filaments), one can summarize that calendered carded webs are challenging substrates with regards to softness.

On the other hand, carded nonwovens have several advantages over spunbonded nonwovens. Carded nonwovens provide material design flexibility to deliver different benefits. For instance, it is possible to include natural fibers such as cotton fibers or other thermally un-bondable natural fibers (e.g., hemp) into the staple fiber blends. Carded nonwovens can also have non-uniform or layered structures which may be desired in certain applications. However, including cotton fibers can also reduce the softness impression of the web. Therefore, it would be desirable to improve the softness of carded calendered nonwoven while keeping its cost advantages.

The use of fatty acid amides to enhance the softness perception of nonwovens has been described in the art. Mechanistically, it is assumed to be due to diffusion ("blooming") of the low molecular weight fatty acid amides to the surface of the nonwoven fiber, where the molecules create a lubricating film (or spots) which gives a slippery feel, e.g., when gliding with a finger across the surface of the nonwoven. Adding copolymer additives to enhance the effect of the fatty acid amid on softness has been described in WO2014/074409 (Han Xu et al.) in the context of spunbond nonwovens. Without wishing to be bound by theory, the inventors believe that the higher crystallinity of carded PP fibers, compared to spunbond PP fibers, reduces the ability of the fatty acid amide to bloom to the surface of carded PP fibers. The higher crystallinity of carded fibers is believed to be due to the additional stretching step in the carded fiber making process. Thus, increasing the softness of carded calendered nonwoven represents different challenges than in the prior art for spunbond nonwovens. Also, spunbond nonwovens typically cannot be mixed with natural fibers because the continuous filaments that are laid down in the spunbond web making process cannot be homogenously blended with the short length natural fibers.

### SUMMARY OF THE INVENTION

The present invention is for a carded calendered nonwoven comprising synthetic fibers having a polypropylene (PP) matrix, an ethylene propylene copolymer and a fatty acid amide. The inventors have found that the carded calendered nonwovens of the invention can provide softness impression comparable to conventional spunbond nonwovens and air-though bonded carded nonwovens. The invention is also for the staple fibers used to make the nonwoven. Natural fibers such as cotton fibers can also be included in the staple fibers.

### BRIEF DESCRIPTION OF THE DRAWINGS

Fig. 1a,b,c show SEM pictures of the calendered side of a nonwoven according to the invention at different magnifications.
Fig. 2a,b,c show SEM pictures of the opposite side of the nonwoven of Fig. 1a,b,c.
Fig. 3a,b,c show SEM pictures of the calendered side of a nonwoven according to the invention at different magnifications further comprising natural fibers.

### DETAILED DESCRIPTION OF THE INVENTION

### Carded calendered nonwoven

The present invention is in a first aspect directed to a carded calendered nonwoven. Carding and subsequent calendering of staple fibers is a known method in the art to make nonwovens (see background section above). The staple fibers used to make the carded nonwovens of the invention comprise synthetic staple fibers comprising a PP matrix, an ethylene-propylene copolymer and a fatty acid amide. The synthetic fibers can be prepared by melting pellets of PP and adding the ethylene-propylene copolymer and the fatty acid amide to form molten mixture which is then extruded in the form of continuous fibers. The continuous fibers are stretched and then cut to make staple fibers of a desired linear density. The linear density for the synthetic fibers may be typically in the range of from 1.5 to 2.5 dtex. The desirable fiber diameter of the synthetic staple fibers may be for example in the range of 14.5 µm to 18.7 µm.

Natural fibers may be mixed with the synthetic staple fibers, if desired. Carding and calendering then take place as is known in the art. The calendering pattern on the calendering roll may be any standard pattern.

Natural fibers may be selected from the group consisting of wheat straw fibers, rice straw fibers, flax fibers, bamboo fibers, cotton fibers, jute fibers, hemp fibers, sisal fibers, bagasse fibers, hesperaloe fibers, miscanthus, marine or freshwater algae/seaweeds and combinations thereof. The natural fibers may for example be selected from the group consisting of cotton fibers, silk fibers, bamboo fibers, or mixtures thereof. The natural fibers may in particular comprise or consists of cotton fibers. Cotton fibers are natural cellulosic fibers that have good liquid acquisition, and good breathability. Incorporating cotton fibers or similar soft natural fibers renders the nonwoven more environmentally friendly as these are renewable resources while improving the fluid handling properties of the nonwoven. This is particularly desirable when using the nonwoven as a topsheet. A carded calendered nonwoven of the invention may typically comprise from 5% to 20% of natural fibers, by weight of the nonwoven, for example 15% by weight of the nonwoven.

In order to increase the tensile strength of the nonwoven, it can be desirable to add a certain percentage e.g., up to 20% by weight of the nonwoven, of a second synthetic staple fiber of a higher fiber linear density (as described in US2012/0066852). This second fiber may have a fiber linear density up to 5 dtex, but has preferably about twice the value of the linear density of the synthetic fiber which represents the majority fiber component. Normally, fibers of a higher diameter have a detrimental impact on softness impression, in the present invention, however, such negative impact can be mitigated.

The bonding area is the area of the nonwoven occupied by the calendering bond points. The bond points can have shapes used in the art, for example rectangular or diamond-shaped, and typically oval shaped. Typically, the bonding area may range from 8% to 30% of the total nonwoven.

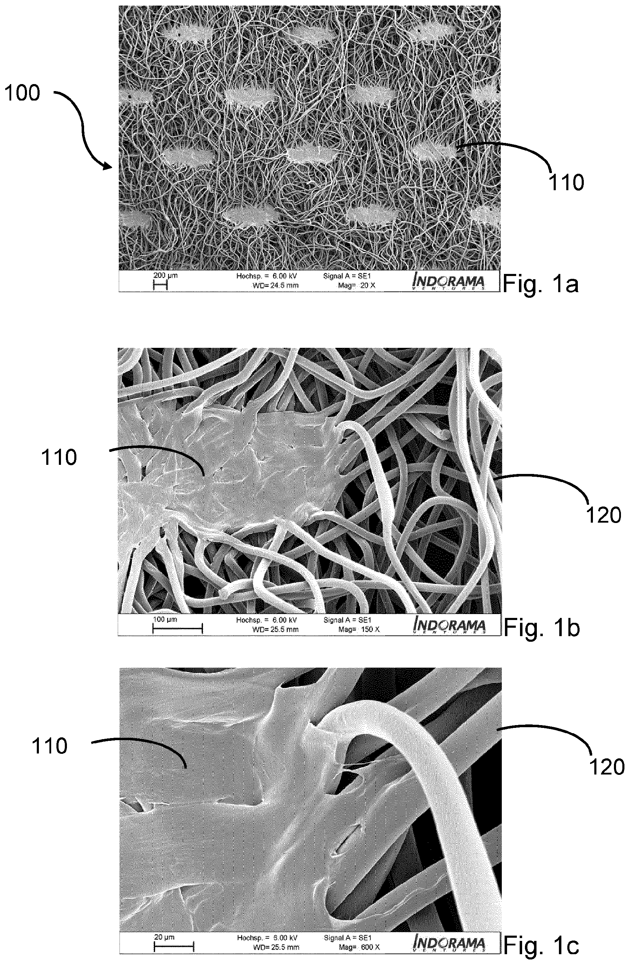

Exemplary values for the bonding pattern include a bonding area in the range of 12 to 14%, a bonding density (number of bond points per area) in the range of about 55/cm² to about 65/cm² and a pitch (distance form center to center of the bond points) is in the range of 1.80 mm to 1.90 mm in both cross direction and machine direction. Such a bonding pattern with oval bond points 100 was used to make the nonwovens illustrated in the SEM pictures of the Figures 1-2.

According to the invention, it was found that forming synthetic staple fibers by adding an ethylene-propylene copolymer and a fatty acid amide to a PP resin matrix can surprisingly increase the softness of the carded calendered nonwoven obtained. The nonwovens of the invention may typically comprise from about 50% to about 100% (all % by weight unless indicated otherwise) of PP based synthetic fibers as defined above. Optionally, other staple fibers can be added to the fiber mix, in particular natural fibers, but also other synthetic fibers, such a mono-component PE or mono-component PP fibers. While it is not excluded that the nonwovens of the invention may also comprise bicomponent fibers, the nonwovens may advantageously be substantially free of bicomponent fibers as these are comparatively costly. "Substantially free" meaning herein comprising less than 10%, in particular less than 5%, more particularly less than 2%, of bicomponent fibers by weight of the nonwoven. The nonwovens of the invention may be completely free of bicomponent fibers.

It has been described in the art (US5,281,378A and EP0,719,879A2) that efficient, high speed spinning and processing of polyolefin fibers such as polypropylene benefit from careful control over the degree of chemical degradation and melt flow rate (MFR) of the spun melt, and a highly efficient quenching step for avoiding both over- or under-quench (i.e. melt fracture or ductile failure) during high speed commercial production. An optimum strength of the bond points can be achieved in the task of bonding PP mono fibers, if a skin-core structure is generated within the PP mono fibers

In such structure the outer layers of the fiber have been partially oxidized during the spinning step, and as a result the PP macromolecules have been cleaved to some extent via an oxygen induced chain scission reaction. This results in an increased "flowability" of the outer skin during the bonding step: the bond points are primarily formed by melting of the weaker skin, while the core of the fiber, which has not been affected by the oxidation, gets less involved in the formation of the bond points.

While not wishing to be bound by theory, the inventors believe that the presence of such skin-core structure in the PP mono fibers to be bonded also enables the adjustment of gentler settings during the bonding step, which again enable an improved softness perception of the web. Reversely, when PP mono fibers without such pronounced skin-core structure necessitate other process conditions during the bonding step (e.g., a higher pressure) to achieve sufficient bond strength, which results in a harsher haptic impression of the web.

It may be advantageous to reduce the spinning linear density to optimize the skin-core effect and obtain optimal thermal bonding performance without sacrificing softness. As a rule of thumb, the spinning linear density (i.e., the fiber linear density of the still unstretched fiber after spinning) may be typically about 60% larger than the final linear density after stretching. It may be advantageous to adjust a spinning linear density which is only about 30% but no more than 40% larger than the final linear density. So, for example in order to end up with a final linear density of 1.7 dtex a spinning linear density of 2.2 dtex is adjusted in the present invention instead of 2.7 dtex, which would have been chosen for PP homopolymer without fatty acid amide or ethylene propylene copolymer added.

In the present invention, an ethylene-propylene copolymer and a fatty acid amide are added to the PP resin used to produce the synthetic staple fibers. The inventors believe that the addition of these two additive components may lead to a thinner skin, and hence decrease the thermo bonding process window. Without wishing to be bound by theory, the inventors believe that the presence of these additive components may inhibit the PP chain scission reaction, which requires the presence of a sufficiently high concentration of tertiary carbon radicals formed in the PP macromolecules. This may result in thermo bond points of reduced strength. A lower line speed in the bonding step may thus be required to cope with the thinner skin. The inventors found that this limitation can be overcome if the synthetic fibers are spun with a relatively narrow first linear density, so that the continuous fiber require less stretching in the stretching step. In this way, a sufficiently thick skin can be generated even in the presence of the two added components but. The skin has advantageously a thickness of 0.1 µm in the unstretched fiber and can be visualized via scanning electron microscopy after a density contrast has been generated via diffusion of a ruthenium salt after salt into the skin. Thus, in the process of making the staple fibers, the first linear density may be no more than 40% larger than the second fiber linear density.

In summary, the inventors have found that a process for making synthetic staple fibers, the synthetic fibers comprising a polypropylene polymer matrix, an ethylene propylene copolymer and a fatty acid amide, the process may comprise the steps of:
- melting a polypropylene polymer matrix, an ethylene propylene copolymer and a fatty acid amide together;
- spinning the molten mixture in continuous fibers having a first linear density
- stretching the continuous fibers directly after the spinning step to a second linear density;
- wherein the first linear density is no more than 40% larger than the second fiber linear density;
- cutting the stretched fibers into staple fibers.

It is known that the addition of natural fibers to a carded nonwoven reduces the mechanical strength of the nonwoven. The inventors found that by application of the above-described measures it is possible to distribute up to 20% of natural fibers homogeneously in z direction across the carded calendered nonwoven, without the need to add a second, mechanically stronger, layer which may be free of natural fibers. Such second layer may have been required to enable sufficient mechanical strength of the total web to enable proper web handling (e.g., winding on and unwinding from rolls, and conversion on diaper production lines) of the carded calendered nonwoven comprising up to 20% of natural fibers. This leaves the design flexibility to either maximize the total amount of natural fibers incorporated in the web without need to introduce a second card (to provide the second layer without natural fibers), or to only position the natural fibers on an upper layer, which is e.g., directed to the user of the hygiene product. Both the absence of a second card in the making process and the absence of an additional support layer contribute to keeping the cost low.

The nonwovens of the invention may preferably achieve a dynamic coefficient of friction (CoF) as measured in machine direction on the calendered side of 0.65 or less (measured according to ASTM D1894 - 01), preferably less than 0.60. The additives and examples of nonwovens according to the invention are described below in further details.

The inventors further believe that the presence of TiO₂ particles may be advantageous to boost the blooming of the fatty acid amide, presumably due to the excluded volume of the inorganic particles. The amount of TiO₂ or other inorganic particles in the synthetic staple fibers may be at least 0.25% by weight of the nonwoven, and up to 1%, for example 0.5% by weight of the nonwoven.

In certain applications, e.g. as use as a topsheet, it may be desirable to increase the hydrophilicity of the nonwoven to make the nonwoven more liquid permeable. This can be achieved by applying a hydrophilic surfactant on the nonwoven, or the individual fibers (hydrophilic spin finish), or by admixing other hydrophilic fibers to the staple fibers before the calendering step.

When processing fibers which are intended for a product where the final application needs to be hydrophobic, it may be advantageous to apply a hydrophobic spin finish. PP is by inherently hydrophobic but during fiber manufacturing, it may be is required to use some lubrication in the texturization process and antistatic agents may also be needed to avoid fibers becoming static and difficult to handle. The antistatic agents are typically hydrophilic of nature and therefore if standard antistatic agent is used, the final nonwoven may not be sufficiently hydrophobic. Special hydrophobic spin finishes are then used to ensure that hydrophobic non-woven can be produced. Examples of hydrophobic melt additives that may be included in the PP mix are selected from hydrophobic silicones, ethoxylated fatty alcohols, lipid esters, in particular glycerol tri-stearate (see e.g., US2016/067118, Hammons et al.).

### Polypropylene polymer matrix

Polypropylene (PP) is a thermoplastic polymer that is widely used in various industries due to its versatile properties. It is a type of plastic that belongs to the polyolefin group. Polypropylene is known for its high strength, good chemical resistance, low density, and availability. The synthetic fibers of the invention comprise a PP-based matrix as main component. The PP polymers that can be used in the present invention as PP-based matrix include PP Homopolymer ("PP-H") and PP Copolymer ("CoPP").

Polypropylene Homopolymer is the most basic form of polypropylene and consists of only propylene monomers. It offers high stiffness, strength, and good chemical resistance. In terms of tacticity, polypropylene (PP) homopolymers can be classified into three main types: Isotactic Polypropylene (i-PP), Syndiotactic Polypropylene (s-PP) and Atactic Polypropylene (a-PP).

Isotactic polypropylene is the most common and commercially available form of PP. In isotactic PP, all the methyl groups (CH3) in the polymer chain are positioned on the same side, resulting in a regular and orderly arrangement of side groups. This arrangement contributes to a high degree of crystallinity, which enhances the polymer's mechanical strength, stiffness, and heat resistance.

Syndiotactic polypropylene has a more irregular arrangement of side groups compared to isotactic PP. In s-PP, the methyl groups alternate in a regular pattern along the polymer chain. The syndiotactic structure leads to a lower degree of crystallinity and decreased polymer chain mobility, resulting in improved transparency and increased flexibility compared to isotactic PP.

Atactic polypropylene has a random arrangement of side groups along the polymer chain. This results in a highly amorphous structure with low crystallinity and poor mechanical properties. Atactic PP is usually produced as a byproduct during PP synthesis and is less commonly used in industrial applications.

The tacticity of polypropylene significantly affects its physical and mechanical properties, such as crystallinity, melting behavior, thermal stability, and flexibility. The specific tacticity of PP can be controlled during the polymerization process by using specific catalysts and reaction conditions.

The PP polymer matrix of the invention is preferably a PP Homopolymer (PP-H), however it is not excluded that a CoPP may also be considered for the PP-based matrix. In that case, the CoPP and the ethylene-propylene copolymer additives are different components of the synthetic fibers. While suitable ethylene-propylene copolymer additives have an amorphous character with an enthalpy of crystallization preferably in the range of 10 J/g to 40 J/g, suitable PP polymer matrix (PP-H or CoPP) have much higher enthalpy of crystallization, typically exceeding 40 J/g, or 50 J/g, e.g. of from 50 J/g to 200 J/g. For the same reasons, the PP-based matrix has typically a melting point which is much higher (at least 20 degree C or at least degree 40 C difference) than the melting point of the ethylene-propylene copolymer additive.

Polypropylene Copolymers comprise at least 50% of propylene monomers as main component, by weight, and one or more other monomers, in particular ethylene. CoPP include random copolymers and block copolymers. Random PP copolymers contain a small amount of comonomer (usually ethylene) randomly distributed within the polymer chain. It exhibits improved clarity, impact resistance, and flexibility compared to homopolymer polypropylene.

Block PP Copolymer are produced by polymerizing propylene such as ethylene and a different comonomer in separate polymerization steps. It combines the properties of both homopolymer and random copolymer polypropylene, offering a balance between stiffness, impact resistance, and transparency. It is commonly used in automotive parts, appliances, and packaging.

Polypropylene has a relatively low density, making it lightweight compared to many other plastics. The density of PP typically ranges from 0.895 to 0.92 grams per cubic centimeter (g/cm³). The melting point of polypropylene is relatively high, typically ranging from 130 to 171 degrees Celsius (266 to 340 degrees Fahrenheit). This property allows PP to be easily melted and molded into various shapes.

Suitable PP resins that may be used to form the PP-based matrix of the invention may in particular have a Melt Flow Index in the range of 4 g/10 min to 40 g/10 min (MFI being measured at 230°C*2.16 kg - ISO 1133, procedure A). This was found to be sufficient flowability for ease of fiber extrusion and resilient for forming a PP-based matrix. Preferred range of the MFI for the PP-based matrix is in the range of from 5 g/10 min to 30 g/10 min, and more preferred range is of from 6 g/10 min to 20 g/10 min. This grade enables on the one hand very good staple fiber spinning behavior and at the same time optimal thermal bonding performance.

An exemplary PP-H is Total PPH 7059, a PP homopolymer with a melt flow index of about 13 g/10 min, which was used in the example below as the resin for making the synthetic PP-based staple fibers.

An exemplary CoPP that may be used as PP-based matrix of the invention is a metallocene random copolymer sold by Total under the Tradename Lumicene^{®} MR10YN9, having a Melt Flow Index of about 10 g/10 min.

### Fatty acid amides

Oleamide and erucamide are fatty acid amides commonly used as slip agent in polyethylene or polypropylene nonwovens. These compounds can be introduced as melt additives in the molten plastic before the making of the nonwovens. Oleamide is derived from mono-unsaturated C18 oleic acid, while erucamide is the amide of C22 mono-unsaturated erucic acid. The molecules of fatty acid amides are known to migrate at the surface of the fibers and thus provide a self-replenishing surface lubrication (effect referred to as "blooming"). This can reduce the coefficient of friction of the nonwovens made from these fibers or filaments. US3,454,519 (Hulse et al.) for example disclose improved textile fibers prepared from isotactic polypropylene resin containing from about 0.01% to about 1.0% by weight of erucamide. Oleamide has a lower basis weight and is known to migrate to the film surface more rapidly than erucamide. Stearamide is also known to reduce the coefficient of friction when used as melt additive to the polymer melt used to make the nonwoven's fibers. Blend of such fatty acids may also be used. Such a blend of stearamide and erucamide is for example disclosed in US6,740,609 (Peng et al.).

Typically, the heat generated during the making of the nonwoven and/or on an absorbent article's converting line is enough for the blooming to occur. The migration of the molecules of fatty acid amides may also be accelerated up by heating the nonwoven at 40-50°C for a few hours if necessary. Sufficient heat can also be provided during the thermo-bonding (calendering) step used in the consolidation of the nonwoven. Typically, the web is wound into rolls within a few seconds after the thermo-bonding step. Typically, under these conditions a temperature of around 40°C is maintained over several hours inside the rolls. It has been found that the insertion of an additional heating step, e.g., by using standard drying equipment (such as for drying surfactant coatings), may accelerate the blooming of erucamide and enables an overall reduction of the coefficient of friction. This can be used for further usage reduction of the fatty acid amide, e.g., erucamide. However, it requires an additional process operation which may not be available at the production line and its costs further energy. So, it needs to be decided in each individual case if to insert an additional heating step for further e.g., erucamide usage level reduction.

Any fatty acid amide melt additives that can reduce the coefficient of friction of the nonwoven may be used. Suitable fatty acid amides include those deriving from a mixture of C12-C28 fatty acids (saturated or unsaturated) and primary or secondary amines. A suitable example of a primary fatty acid amide includes those derived from a fatty acid and ammonia as illustrated in [1]. 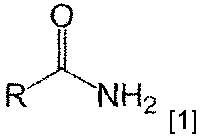 where R has a number of carbon atoms ranging from 11 to 27, in particular from 16 to 22 fatty acid. The fatty acid amide may be comprised of a single compound, e.g., erucamide, or a mixture thereof. The synthetic staple fibers may typically comprise at least 0.05%, preferably from about 0.1% to about 2%, of the fatty acid amide or mixture thereof, by weight of the nonwoven.

The inventors found that the presence of a fatty acid amide at the surface of the PP fibers and at the surface of the bond points provide a softness impression to the carded calendered nonwoven. The surface concentration of the fatty acid amide may reach values above 0.023 on the bond points, and above 0.030 on the PP fibers (as measured with the TOF SIMS normalized intensity method described below). The carded calendered nonwovens of the invention have a soft and textile like perception, in comparison to reference standards of soft nonwovens. Without wishing to be bound by theory, the inventors believe that a certain minimum surface concentration of erucamide is required so that a bilayer of fatty acid amide molecules can form on the surface, in which the lower layer is anchored via the nonpolar "tail" of the molecule in the PP matrix, with its polar "heads" (the amide group) sticking out of the surface. The upper layer of the fatty acid amide bilayer is only loosely attached via hydrogen bonds to that lower layer. The loosely bonded upper layer can freely slide when one glides with a finger over the surface, which provides the impression of softness.

### Ethylene-propylene copolymer(s)

The synthetic staple fibers comprise in addition to the PP polymer matrix and fatty acid amid at least one ethylene-propylene copolymer as additive. Suitable ethylene-propylene copolymers are formed from olefin monomer units, i.e., propylene with ethylene copolymerized together. The copolymers are preferably metallocene-technology based, and thus are produced using a metallocene catalysts, but other catalysts such as Ziegler Natta can also be used. Metallocene-technology based polymers typically have a regular spatial repeat monomer unit distribution and a narrow molecular weight distribution, as is known in the art.

The ethylene-propylene copolymers may typically comprise at least 50% by weight of propylene units, in particular at least 60%, or at least 70%, or at least 80% by weight. The remaining monomers are ethylene monomers, and optionally other alpha olefin monomers that may be present in the co-polymers, for example 4-methyl-1-pentene, pentene-1, 2-methylpentene-1, 3-methylbutene-1, heptene-1, dimethylpentene-1, trimethylbutene-1, ethylpentene-1, methylpentene-1, trimethylpentene-1, methylethylpentene-1, 1-octene, diethylbutene-1, propylpentane-1, decene-1, methylnonene-1, nonene-1, trimethylheptene-1, methylethylbutene-1, dodecene-1, and hexadodecene-1, and combinations thereof. The exact monomer distribution is typically published by the copolymer material supplier, but can also be determined by a suitable method, such as nuclear magnetic resonance or infrared spectroscopies.

The ethylene-propylene copolymer is preferably comprised of a single material as defined above, as this simplifies the compounding and formulation of the fibers, but it is not excluded that the copolymer may also be a blend of individual copolymer materials falling under this definition.

The ethylene-propylene copolymer (or mixtures therefore if a blend is used) may be comprised in the range of from 5% to 40% by weight of the synthetic fibers, in particular from 10% to 20% by weight of the polypropylene synthetic fiber composition.

Nonlimiting examples of commercially available ethylene-propylene copolymers are Affinity EG 8200G, Engage 8200, Infuse 9817, Vistamaxx 3000, Vistamaxx 6102, Vistamaxx 6202, Vistamaxx 6502, VERsify 4200, VERsify 4301.

The ethylene-propylene copolymers may comprise greater than 80 wt.% of polypropylene units with isotactic stereochemistry and random ethylene distribution. Examples of such copolymers are commercially available as the Vistamaxx series from ExxonMobil, for example Vistamaxx 6202, Vistamaxx 6502 and Vistamaxx 7050. These copolymers are primarily composed of isotactic propylene repeat units with random ethylene distribution, produced using a metallocene catalyst technology. They are sold as pellets which can be molten with the PP matrix pellets before spinning the synthetic fibers.

Among the various commercially available ethylene-propylene copolymers, certain combination of molecular weight and enthalpy of crystallization of the ethylene propylene copolymer are believed to enhance the blooming of the fatty acid amide from the carded fibers, and from the calendered nonwoven made thereof.

Without wishing to be bound by theory, the inventors believe that the presence of an ethylene-propylene copolymer as defined by the present invention reduces the compatibility of the fatty acid amide with the PP host matrix and therewith increases the driving force for blooming of the fatty acid amide onto the surface.

The synthetic fibers of the present invention do not require a further polymer additive beyond the ethylene propylene copolymer in order to enhance the softness impression by modifying the bulk mechanical properties of the fiber, e.g. by decreasing the flexural modulus. In particular, the synthetic fibers of the present invention may be free of a ductile propylene polymer with a melt flow index above 1000 grams per 10 minutes, as determined at a load of 2160 grams and at 230°C in accordance with ASTM D1238-13.

The ethylene-propylene copolymer additives suitable in the present invention are relatively amorphous, which may be linked to a relatively low isotactic character of the propylene functional portions of the copolymers.

As detailed in the example section below, the enthalpy of crystallization of the ethylene-propylene copolymer may be above 10 J/g, preferably above 15 J/g (as measured by Enthalpy of Crystallization Test Method). The enthalpy of crystallization may typically reach a value of up to 40 J/g. The enthalpy of crystallization of the ethylene-propylene copolymer is preferably in the range of 10 J/g to 40 J/g.

In addition, or alternatively, the molecular weight of the ethylene-propylene copolymer may be above 100,000 g/mol, preferably above 140,000 g/mol (as measured by the Peak Molecular Weight (Mp) Measurement Method described below). The Peak Molecular Weight may typically have a value of up to 800,000 g/mol.

Table 2 in the experimental section below reports the values for the enthalpy of crystallization and molecular weight of different Vistamaxx additives that may be used in the present invention.

### Optional components

Optional components may be further added to the synthetic fibers as is known in the art, in particular pigments, plasticizer, UV stabilizer, antioxidant, brightener, colorant.

### Test methods

### Peak Molecular Weight (Mp) Measurement Method

The peak molecular weight is determined using a gel permeation chromatography (GPC) method. GPC is a well-known method wherein polymers are separated according to molecular size, the largest molecule eluting first. The peak molecular weights referred to herein can be determined with gel permeation chromatography (GPC) using polystyrene calibration standards, according to ASTM D5296. The molecular weight of any polymer or unknown polymer measured using GPC so calibrated is the styrene equivalent molecular weight, which herein is defined as the "peak molecular weight". Suitable solvents and temperatures are employed with GPC in order to achieve adequate molecular weight separation and resolution.

### Enthalpy of Crystallization Test Method

The Enthalpy of Crystallization of a polymer is determined using ASTM D3418-15 (*"Standard Test Method For Transition Temperatures And Enthalpies Of Fusion And Crystallization Of Polymers By Differential Scanning Calorimetry"*) with the following additional guidance. Dry nitrogen is used as the purge gas in the differential scanning calorimeter (DSC). The rate of increase of temperature in the DSC is 10 °C/min, and the rate of decrease of temperature in the DSC is 1 °C/min. The mass-normalized enthalpy of crystallization is calculated as specified in section 11.4 based on the curve corresponding to decreasing temperature (at 1° C./min) and is reported as the "Enthalpy of Crystallization" in units of joules per gram (J/g) to the nearest 0.1 J/g.

### Dynamic Coefficient of Friction (D-CoF)

The Dynamic Coefficient of Friction is measured using ASTM Method D 1894-01. It is measured in machine direction of the nonwoven. All testing is performed in a room where the temperature is controlled at about 21°C ± 2 C° and 50% ± 10% relative humidity with all samples and nonwoven targets conditioned under the same conditions for at least two (2) hours prior to testing.

The calendered surface is directed toward the target surface.

The test is repeated for a total of ten (10) replicates. Average values are calculated separately for Static CoF for each of the specified targets and reported to the nearest 0.01 units.

### TOF-SIMS Normalized Intensity Method

The TOF-SIMS Normalized Intensity (as measure of the fatty acid amide surface concentration) is determined by using a Time-of-Flight Secondary Ion Mass Spectrometer (TOF-SIMS) (TOF.SIMS 5, M 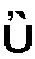 nster, Germany). TOF-SIMS provides molecular, elemental, and isotopic information from the outer most, 10-20 Å, of the sample surface with high chemical sensitivity. Principle operation of TOF-SIMS: A pulsed energetic primary ion beam bombards the sample surface to generate secondary ions in a high vacuum environment. The secondary ions are extracted into a TOF mass spectrometer, which consists of a field-free drift tube of a known length, where the ions travel to the detector. The flight time of a given ion will vary according to its mass and lighter ions will reach the detector before heavier ions. Data generated from this technique is a mass spectrum of either positive or negative polarity, where m/z (m: secondary ion mass, z: charge) is plotted against counts of the detected ion from the surface. TOF-SIMS is only quantitative with reference standards due to the matrix effect; however, relative concentration comparisons can be made by comparing species of similar concentrations in the same matrix.

The samples were prepared at room temperature with standard relative humidity (between 30% and 40%). The samples were handled in a manner to reduce surface contamination and mounted to the sample holder with double-sided sticky tape. Sample size of the non-woven material was approximately 2 cm x 2 cm.

TOF-SIMS measurements were made by rastering a 30 keV Bi3+ primary ion beam over 500 um x 500 um area with low energy electrons for charge compensation in positive polarity mode. The total ion dose was for the analysis was below the static limit. The evaluation is explained at the example of the fatty acid amide erucamide: Based on spectra from an erucamide reference material, it was determined that +m/z 338 (M+H+, M=C22H43NO) is the unique mass that corresponds to erucamide. The TOF SIMS normalized intensities were calculated by normalizing the erucamide peak area (+m/z 338) to the total ion intensity for each analysis. Measurements were made at randomly selected nonwoven (non-bonded) and bonded areas. TOF SIMS normalized intensities were based on the average of five measurements for each area, nonwoven and bonded. Data processing was completed with Surface Lab 7 (IONTOF, M 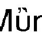 nster, Germany). Based on this method, TOF-SIMS Normalized Intensities were ascertained at the surface of the fibers (non-bonded) and at the surface of the bond points (bonded areas) of the material.

### Examples

Different carded calendered nonwovens were made using staple PP-based fibers. The control nonwovens comprised 100% PP-H staple fibers and did not include a fatty acid amid, nor an ethylene-propylene copolymer component. The staple fibers used in control 1 and control 2 had different linear density.

Example 1 and example 2 are carded, calendered nonwovens consisting of synthetic staple fibers according to the invention. The synthetic fibers comprised a PP-H based matrix (Total PPH 7059), 1% erucamide and 15% of different ethylene-propylene copolymers, by weight of the staple fibers, as summarized in the Table 1 below.

Example 3 additionally comprised 15% cotton staple fibers, by weight of the nonwoven. Also, in example 3, the PP-based synthetic fibers comprised a lower amount of erucamide (0.3% erucamide by weight of the synthetic fibers) compared to examples 1 and 2.

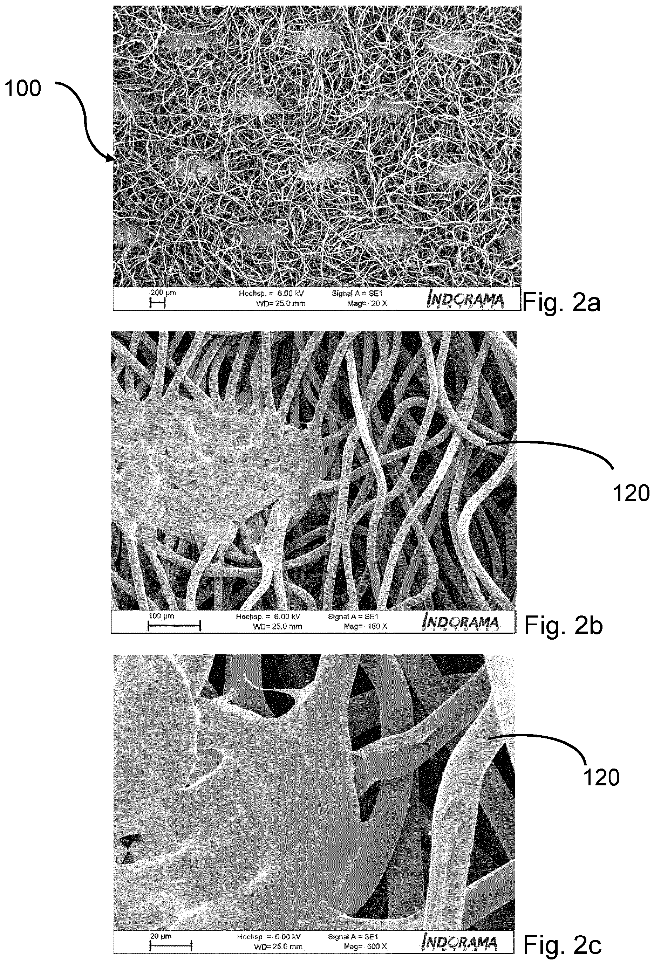

All synthetic staple fibers used in the examples and control samples contained 0.5% TiO₂. After the carding, the nonwoven was calendered between a bottom smooth roll and a patterned top roll with the following settings:
Temperature on rollers: 135 °C on both smooth and patterned roller
Roller pressure: 40 bar
Line speed: 50 m/min
Contact time: 0.041 s
Fig. 1a,b,c show SEM pictures at different magnification of the calendered (top) surface of the nonwoven 100 corresponding to example 2. Fig. 2a,b,c shows the corresponding opposite surface of the nonwoven of example 2 (bottom surface). In examples 1-2 the calendering pattern consisted of oval bond points 110. The individual staple synthetic fibers 120 are visible in Figs. 2-3.

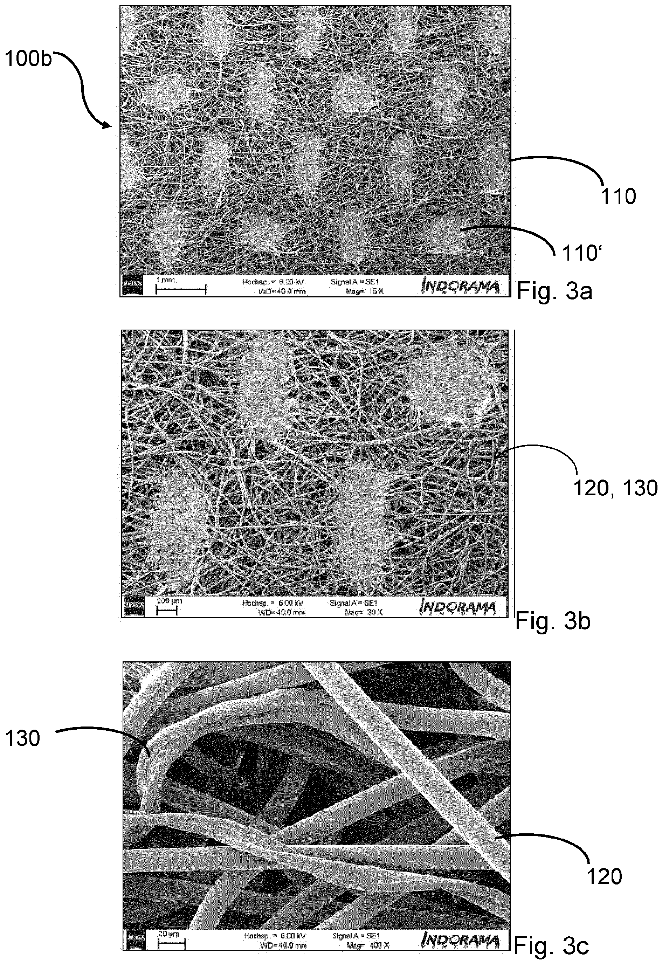

Fig. 3a,b,c show SEM pictures at different magnification of the calendered (top) surface of the nonwoven 100b corresponding to example 3. In this example, the calendering pattern consisted of a combination of oval bond points 110 and circular bond points 110'. A mix of synthetic staple fibers 120 and natural cotton fibers 130 were carded and calendered. The different fibers are recognizable in Fig. 3c.

The Erucamide surface concentration was evaluated on the bonded area 100 and the non-bonded area using the TOF SIMS normalized intensity method. The Dynamic Coefficient of Friction (D-COF) of the nonwovens was also measured.

**Table 1**

| NW Sample | Erucamide dosage | Ethylene-propylene copolymer grade, dosage 15% | Cotton content | Fiber Linear density [dtex] | Erucamide Surface Concentration TOF SIMS Normalized Intensity [10⁻²] | | D-COF |
|---|---|---|---|---|---|---|---|
| | | | | | Bonded Area | Non-bonded Area | |
| Control 1 | None | none | 0% | 1.0 | 0 | 0 | 0.69 |
| Control 2 | None | none | 0% | 1.7 | 0 | 0 | 0.68 |
| Example 1 | 1% | Vistamaxx 6202 | 0% | 1.7 | 1.83 | 2.53 | 0.54 |
| Example 2 | 1% | Vistamaxx 7050 | 0% | 1.7 | 2.35 | 3.04 | 0.49 |
| Example 3 | 0.30% | Vistamaxx 7050 | 15% | 1.7 | NA | NA | 0.54 |

The staple fibers used in control 1 and 2 had different linear density. The softness impression was rated in a sensory panel in comparison to a reference standard. The smaller fiber diameter used in control 1 did not result in a lower COF or an enhanced softness impression of the carded calendered nonwoven made from these fibers.

In control 2 and examples 1 to 3 a fiber linear density of 2.2 dtex was adjusted for spinning: the final fiber linear density of 1.7 dtex was achieved through the stretching step.

Examples 1-3 were found softer than the control examples as indicated by the lower D-COF values. The lowest D-COF and best softness impression was achieved in example 2. This was confirmed via measurements of the erucamide surface concentration by the TOF-SIMS method. Without being wished to be bound by theory, it is believed that the superior softness is enabled by a higher extent of blooming of erucamide to the surface of the nonwoven.

The erucamide surface concentration was measured for example 3 because of the presence of surface cotton fibers besides the polypropylene fibers at the surface of the nonwoven. However, a sufficient softness could still be achieved, even with a reduced amount of erucamide. Example 3 may be used for example as an outer cover nonwoven in a diaper. Example 3 also had sufficient strength to enable web handling (and at the same time sufficient softness), despite the presence of natural fibers (homogeneously distributed across z direction).

If desired, a hydrophilic surfactant is applied onto the nonwoven of any of the examples, for example if it is desired to use the nonwoven as a topsheet in a diaper.

The impact of the ethylene propylene copolymers grade was tested by making staple PP-fibers comprising 1% erucamide and 15% ethylene propylene copolymers having different grade as indicated above. The softness of the resulting carded calendered nonwovens was assessed by an expert panel as follows.

**Table 2**

| Ethylene-propylene Copolymer | Crystallization Enthalpy [J/g] | Molecular Weight [g/mol] | Softness grading of resulting carded calendered NW |
|---|---|---|---|
| Preferred Requirement: | = or > 10 | >100,000 | |
| Vistamaxx 6202 | 7.8 | 146,746 | good |
| Vistamaxx 6102 | 8.7 | 310,656 | good |
| Vistamaxx 7050 | 16.0 | 146,781 | best |
| Vistamaxx 8880 | 32.3 | 23,351 | less good |

While not wishing to be bound by theory, the inventors believe that ethylene-propylene copolymers grade having 1) an enthalpy of crystallization of at least 10 J/g, preferably above 15 J/g, and/or 2) a molecular weight of the ethylene-propylene copolymer above 100,000 g/mol, preferably above 140,000 g/mol, are more effective in boosting sufficient blooming of erucamide at the surface of the nonwoven.

### Misc

As used herein, the terms "comprise(s)" and "comprising" are open-ended; each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essentially of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "preferably", "advantageously", "in particular" and the likes also qualify features which are not intended to limit the scope of the claims unless specifically indicated to do so.

Unless indicated otherwise, the description and claims refer to the absorbent core and article before use (i.e. dry, and not loaded with a fluid) and conditioned at least 24 hours at 21 °C +/- 2 °C and 50 +/- 5% Relative Humidity (RH).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A carded calendered nonwoven (100) comprising synthetic staple fibers (110) and optionally natural fibers (120), wherein the synthetic staple fibers comprise a polypropylene polymer matrix, an ethylene-propylene copolymer and a fatty acid amide.

2. The carded calendered nonwoven according to claim 1, comprising from about 5% to about 20% natural fibers, by weight of the carded calendered nonwoven.

3. The carded calendered nonwoven according to any of the preceding claims, wherein the enthalpy of crystallization of the ethylene-propylene copolymer is at least 10 J/g, preferably in the range of from 10 J/g to 40J/g, the enthalpy of crystallization being measured as indicated in the Enthalpy of Crystallization Test Method indicated herein.

4. The carded calendered nonwoven according to any of the preceding claims, wherein the ethylene-propylene copolymer has a peak molecular weight above 100,000 g/mol, as measured by the Peak Molecular Weight Measurement Method indicated herein, preferably wherein the ethylene-propylene copolymer has a peak molecular weight above 140,000 g/mol.

5. The carded calendered nonwoven according to any of the preceding claims, wherein the synthetic staple fibers comprise from 5% to 25%, preferably from 10% to 20%, of the ethylene-propylene copolymer or mixtures thereof, by weight of the staple synthetic fibers.

6. The carded calendered nonwoven according to any of the preceding claims, wherein the fatty acid amide is selected from Oleamide, Erucamide, Stearamide and mixtures thereof.

7. The carded calendered nonwoven according to any of the preceding claims, wherein the synthetic staple fibers comprise at least 0.05% of the fatty acid amide(s) or mixture thereof, by weight of the synthetic staple fibers, preferably from about 0.1% to about 2% of the fatty acid amide(s) by weight of the synthetic staple fibers.

8. The carded calendered nonwoven according to any of the preceding claims, wherein the carded calendered nonwoven further comprises a surfactant to increase its hydrophilicity.

9. The carded calendered nonwoven according to any of the preceding claims, wherein the carded calendered nonwoven is substantially free of bicomponent fibers.

10. The carded calendered nonwoven according to any of the preceding claims, wherein the basis weight of the carded calendered nonwoven is in the range of from about 10 g/m² to about 40 g/m².

11. The carded calendered nonwoven according to any of the preceding claims, wherein the carded nonwoven has a dynamic coefficient of friction as measured in machine direction of 0.65 or less, as measured according to ASTM D1894 - 01.

12. A process for making a carded calendered nonwoven according to any of the preceding claims, comprising the steps of:
providing staple synthetic fibers, the synthetic fibers comprising a polypropylene polymer matrix, an ethylene-propylene copolymer and a fatty acid amide;
optionally mixing the synthetic fibers with natural fibers;
carding the staple fibers into a web;
calendering the web between two rolls, thus forming bonding points on the web.

13. Staple fibers comprising synthetic staple fibers (110) and optionally natural fibers (120), wherein the synthetic staple fibers comprise a polypropylene polymer matrix, an ethylene-propylene copolymer and a fatty acid amide.

14. A process for making synthetic staple fibers according to the preceding claim,
wherein the synthetic staple fibers are obtained by the process of:
melting the polypropylene polymer matrix, the ethylene-propylene copolymer and the fatty acid amide together to obtain a molten mix;
spinning the molten mix into continuous synthetic fibers;
cutting the continuous synthetic fibers into staple synthetic fibers;
optionally mixing the synthetic staple fibers with natural fibers.

15. The process according to the preceding claim, wherein the synthetic staple fibers are obtained by the process of:
melting the polypropylene polymer matrix, the ethylene-propylene copolymer and the fatty acid amide together to obtain a molten mix;
spinning the molten mix in continuous fibers having a first linear density;
stretching the continuous fibers directly after the spinning step to a second linear density, wherein the first linear density is no more than 40% larger than the second fiber linear density;
cutting the stretched continuous fibers into staple synthetic fibers.
